# EUROPEAN PATENT SPECIFICATION

(11) **EP 2 668 292 B1**
(45) Date of publication and mention of the grant of the patent: **22.03.2017**
(21) Application number: 12705723.0
(22) Date of filing: 24.01.2012
(51) Int. Cl.: C12Q 1/68, G06F 19/22

(54) **DETECTION OF INFECTION BY A MICROORGANISM USING SMALL RNA SEQUENCING SUBTRACTION AND ASSEMBLY**
ERKENNUNG EINER INFEKTION DURCH EINEN MIKROORGANISMUS ANHAND VON SRNA-SEQUENZIERUNGSSUBTRAKTION UND ANORDNUNG
DÉTECTION D'INFECTION PAR UN MICRO-ORGANISME PAR SOUSTRACTION ET REGROUPEMENT DE SÉQUENCES DE PETITS ARN

(30) Priority: 26.01.2011 US 201161436198 P
(43) Date of publication of application: 04.12.2013
(73) Proprietor: Ramot at Tel-Aviv University Ltd., 61392 Tel Aviv (IL)
(72) Inventor: ISAKOV, Ofer, 70300 Be'er Yakov (IL); MODAI, Shira, 47203 Ramat Hasharon (IL); SHOMRON, Noam, 46305 Herzelia (IL)
(74) Representative: Becker Kurig Straus
(86) International application number: PCT/IL2012/050026
(87) International publication number: WO 2012/101643

(56) References cited:
- WO-A2-2005/033271
- US-A1- 2002 015 955
- KREUZE J F ET AL: "Complete viral genome sequence and discovery of novel viruses by deep sequencing of small RNAs: A generic method for diagnosis, discovery and sequencing of viruses", VIROLOGY, ACADEMIC PRESS,ORLANDO, US, vol. 388, no. 1, 25 May 2009 (2009-05-25), pages 1-7, XP026087983, ISSN: 0042-6822, DOI: 10.1016/J.VIROL.2009.03.024 [retrieved on 2009-04-23]
- WU QINGFA ET AL: "Virus discovery by deep sequencing and assembly of virus-derived small silencing RNAs", PROCEEDINGS OF THE NATIONAL ACADEMY OF SCIENCES OF THE UNITED STATES OF AMERICA, vol. 107, no. 4, January 2010 (2010-01), pages 1606-1611, XP002676034, ISSN: 0027-8424
- ISAKOV OFER ET AL: "Pathogen detection using short-RNA deep sequencing subtraction and assembly", BIOINFORMATICS (OXFORD), vol. 27, no. 15, August 2011 (2011-08), pages 2027-2030, XP002676035,
- LAURENT FARINELLI: 'Recent developments in small RNA analyses using massively parallel sequencing', [Online] 07 May 2010, XP055255354 Retrieved from the Internet: <URL:https://www.fasteris.com/pdf/2010-05-0 7_Fasteris_Bern_Seminar.pdf> [retrieved on 2016-03-04]

## Description

### FIELD OF THE INVENTION

The present invention relates to methods and systems for the detection of infection of a subject by a microorganism, by using small RNA derived sequences.

### BACKGROUND OF THE INVENTION

Early and accurate detection of human pathogen infection is critical for treatment and therapeutics. Most pathogen detection methods such as, for example, PCR amplification, or microarrays rely on prior knowledge of the exact sequence of the potential pathogen, epidemiology of the pathogen infection, or the ability to cultivate it (for example, by microbial cultures). However, these detection methods are time consuming and are unreasonable in many cases, in particular when the pathogen causing the disease or symptoms is unknown.

An alternative detection technique includes the sequencing of infected cells and comparing the sequences to a reference pathogen library for identification (for example, MacConaill and Meyerson (2008)). An infected tissue may contain nucleic acids from both the host and the infecting microorganism. It has been shown that filtering long sequence reads from a transcript sample against the human genome and analyzing the non-human reads has the potential of identifying non-human pathogen genes (Xu, *et.al.* (2003)).

In another example, US patent No. 6,996,477, directed to computational subtraction method, provides a method and system for performing computational subtraction to detect microbes within a host organism.

Nevertheless, in order to successfully detect and identify minute quantities of microorganism which is infecting a subject, there is a need to overcome disadvantages of such prior art methods, which make use of thousands of long sequence reads (>200bp) of larger nucleic acid molecules. When using larger sequences of DNA molecules to detect pathogen infection of a host, the background reading can be very high and the detection level can be low. Since host DNA quantity is usually several orders of magnitude higher than the pathogen DNA molecules, the relative level of nucleic acids that belong to the pathogen are very low as compared to those of the host. Furthermore, pathogens may be somewhat dormant and present/express very small amount sequences of themselves, which makes it even harder to identify their presence. Additionally, when using large/long nucleic acid molecules for sequencing, splicing events of these longer transcripts may occur and thus might complicate the alignment process. Because of these disadvantages, such methods are usually applicable for certain pathogens, in particular those with higher expression levels and only qualitative and not quantitative information can be deduced.

There is thus a need in the art for a robust, more sensitive, accurate, reliable, fast, unbiased and quantitative method, which is further reproducible and comparative and allows the specific, accurate, unbiased and rapid detection of a microorganism infection of a subject.

### SUMMARY OF THE INVENTION

The present invention is defined by claims 1-14. Disclosed herein is a method for an early and accurate detection and/or identification of infection of a subject by a microorganism. The identification of the infectious microorganism is obtained by the subtraction and assembly of small RNA molecules extracted from a sample derived from an infected subject.

According to some embodiments, the disclosure is based in part on the surprising finding that small RNA molecules and sequences derived therefrom can be successfully used and applied in a method for the detection and identification of infection of a subject by a microorganism. The small RNA molecules utilized by the method can be coding and/or non-coding small RNA molecules. The use of small RNA molecules derived sequences overcomes disadvantages of prior art methods. Use of small RNA sequences for microorganism detection and/or identification, rather than, for example, the commonly used cDNA and expressed sequence tags (ESTs) maximizes the microorganism to subject nucleotide ratio which is a major breakthrough in the identification and analysis of relatively small organisms with minimal expression level. Specific short RNA extracts provide higher microorganism-to-subject ratio than the use of DNA or large RNA extracts, since, as explained herein above, the subject's DNA/RNA content is usually several orders of magnitude higher than the microorganism DNA/RNA content. The increase in the microorganism small RNA quantity is achieved due to a higher RNA degradation rate in the microorganism as compared to the host, as well as the presence of fragmented microorganism sequences (due, for example, to RNA interference (RNAi) mechanisms and other RNA degradation processes), leading to prevalent microorganism RNA of lower molecular weights and smaller/shorter lengths. Further, unambiguous mapping of short RNAs to the reference subject genome is preferred to circumvent splicing events of longer transcripts, since the proportion of reads spanning splice junctions is minute. Additionally, by utilizing short RNA molecules, the highly abundant RNA molecules originating from the subject (such as, for example, rRNA and tRNA) that may mask the overall reads and add unwanted background are excluded.

Also provided herein is a solution for the confounding problem of multiple microorganisms alignment and the inconclusive identification of the infectious microorganisms by using sequencing data derived from short sequence reads in general and from small RNA molecules in particular.

According to yet further embodiments, and as opposed to prior art methods, the method for the detection and identification of infection of a subject by a microorganism may be used as a qualitative and/or as a quantitative method.

According to some embodiments, there is thus provided a novel method for the detection of infection of a subject by a microorganism, using short sequencing reads generated by deep sequencing of short-RNA extracts obtained from a biological sample of the subject. According to some embodiments, the method comprises the steps of: (i) isolating a fraction of small RNA molecules from a biological sample of the subject; (ii) alignment of the short reads against the subjects' reference genome; (iii) subtraction and assembly of the remaining unmapped reads; and (iv) categorization and identification of infection by the microorganism based on nucleic acid databases. In some embodiments, the subject is a mammal, such as a human. In some embodiments, the subject is a plant. In some embodiments the microorganism is a virus, a bacteria, a fungi, a parasite, or the like, or combinations thereof.

According to some embodiments, there is provided a method for the detection and identification of infection of a subject by a microorganism, the method comprises the steps of extracting small RNA from biological sample (such as, for example, cells/tissue) of the subject; determining the sequences of the cDNA molecules derived from the small RNAs (thereby producing short reads); alignment of the short reads against the subject reference genome; identification of the non-subject sequences (unmapped reads) and identify to which microorganism it aligns; apply de-novo sequencing (sequence assembly) to produce longer consensus sequences (contigs) from the short read sample; comparing the contigs to known microorganisms references (by performing alignments); and identify the infectious microorganism.

According to some embodiments, there is provided a method for the detection of infection of a subject by a microorganism, the method comprising: isolating a fraction of small RNA molecules from a biological sample of the subject; aligning of nucleic acid sequences derived from the small RNA molecules against a database comprising the subject nucleic acid genome sequence; subtracting and assembling unmapped sequences to produce contigs; and identifying the microorganism based on alignment of the contigs against databases comprising nucleic acid sequences.

According to some embodiments, the isolated small RNA molecules are further reverse transcribed and polymerase chain reaction (PCR) amplified to obtain short cDNA products. According to additional embodiments, the cDNA products are further sequenced, thereby producing short reads. According to additional embodiments, the short reads are aligned against the subjects' reference genome and the mapped reads are subtracted. According to additional embodiments, the method further comprises the step of applying de-novo sequencing (sequence assembly) to produce longer consensus sequences (contigs) from the short unmapped reads.

According to some embodiments, the small RNA molecules are coding RNA molecules or fragments thereof. According to some embodiments, the small RNA molecules are non-coding RNA molecules or fragments thereof. According to some embodiments, the small RNA molecules comprise piRNA, siRNA, miRNA, shRNA, snRNA, snoRNA, and/or any other small RNA molecule that is a degradation product of a larger RNA molecule. According to some embodiments, the small RNA molecules have a length of about 10-100 nucleotides. For example, the small RNA molecules have a length of about 10-50 nucleotides. For example, the small RNA molecules have a length of about 20-50 nucleotides. For example, the small RNA molecules have a length of about 20-35 nucleotides. For example, the small RNA molecules have a length of about 22-30 nucleotides.

According to further embodiments, the contigs have a length of about 20-500 nucleotides. For example, the contigs have a length of about 25-300 nucleotides. For example, the contigs have a length of about 50-150 nucleotides. For example, the contigs have a length of about 65-85. For example, the contigs have a length of about 85-105 nucleotides.

According to additional embodiments, the databases comprising the subject's nucleic acid genome sequence comprise at least a partial sequence of the subject's nucleic acid genome. According to further embodiments, the databases comprising the subject's nucleic acid genome sequence comprise the entire sequence of the subject's nucleic acid genome. According to some embodiments, the databases comprising nucleic acid sequences may be selected from, but are not limited to: GenBank CDS (Coding sequences database), PDB (protein database), SwissProt database, PIR (Protein Information Resource) database, PRF (protein sequence) database and EMBL Nucleotide Sequence database, collection of RNA families (Rfam) database, or any other sequence and information databases, either known today or to be developed in the future.

According to some embodiments, the alignment of the reads is performed by computational tools. The computational tools may be selected from, for example, but not limited to: deep sequencing small RNA analysis pipeline (DSAP), miRTools, miRExpress, miRNAkey, ncRNA read and analyze (RandA), Burrows-Wheeler Aligner (BWA) alignment software (BWA), Stampy, Basic Local Alignment Search Tool (BLAST), BLAT, Novoalign, Bowtie, Mosaik, Blat-like Fast Accurate Search Tool (BFAST), Micro Read Fast Alignments Search Toll (mrFAST), Mapping and Assembly with Quality (MAQ) or the like, or any other suitable computational tool, either known today or to be developed in the future.

According to some embodiments, the microorganism is, for example, a bacteria, a virus, a fungi, a parasite, or combinations thereof. In exemplary embodiments, the bacteria is, for example, a gram negative bacteria, a gram positive bacteria, or both. In other exemplary embodiments, the virus is selected from dsDNA viruses, ssDNA viruses, dsRNA viruses, (+)ssRNA viruses (+)sense RNA, (-)ssRNA viruses (-)sense RNA, ssRNA-RT viruses (+)sense RNA with DNA intermediate in life-cycle, sDNA-RT viruses or combinations thereof. In additional exemplary embodiments, the fungi is, for example, yeast, mold, or both. In yet further exemplary embodiments, the parasite is, for example, protozoa, helminths, or both. In further exemplary embodiments, the microorganism is a cancer causing microorganism. In yet further exemplary embodiments, the microorganism is a microorganism causing Acquired Immune Deficiency Syndrome (AIDS).

According to some embodiments, the subject is a human, an animal, or a plant.

According to further embodiments, the small RNA molecules may be obtained from a biological sample of the subject, wherein the biological sample comprises a cell, a tissue, a body fluid, or combinations thereof.

According to further embodiments, at least one of the steps of the method may be performed using a computational system/unit/device.

According to additional embodiments, the method may further comprise a step of calculating a confidence score which is indicative of the confidence level that a microorganism is infecting the subject. According to some embodiments, the confidence score may further be correlated/indicative to the level of infection by the microorganism.

According to further embodiments, the method may be quantitative, qualitative, or both.

According to further embodiments, there is provided a system for detecting infection of a subject by a microorganism, the system comprising: a sequencing device configured to determine nucleic acid sequences derived from small RNA molecules obtained from a biological sample of a subject; and a computing unit configured to: a) align the nucleic acid sequences against a database comprising the subject nucleic acid genome sequence; b) subtract and assemble unmapped sequences to produce contigs; and c) identify a microorganism based on alignment of the contigs against databases comprising nucleic acid sequences. In some embodiments, the computing unit is further configured to calculate a confidence score indicative of the confidence level that a microorganism is infecting the subject. In some embodiments, the sequencing device is a deep sequencer. In some embodiments, the computing unit may be selected from any suitable computational device or computational system.

According to additional embodiments there is provided a non-transitory computer-readable medium having stored therein instructions for detecting of infection of a subject by a microorganism, wherein the instructions, when executed by the computer, cause the computer to: a) align nucleic acid sequences derived from small RNA molecules obtained from a biological sample of a subject, against a database comprising the subject nucleic acid genome sequence; b) subtract and assemble unmapped sequences to produce contigs; and c) identify a microorganism based on alignment of the contigs against databases comprising nucleic acid sequences.

### BRIEF DESCRIPTION OF THE FIGURES

Exemplary embodiments are illustrated in referenced figures. Dimensions of components and features shown in the figures are generally chosen for convenience and clarity of presentation and are not necessarily shown to scale. The figures are listed below.
**Figure 1** is a flow chart of a method for the detection of infection by a microorganism, according to some embodiments;
**Figure 2** is a pictogram of an agarose gel of cDNA fragments showing the presence of mycoplasma in combination with the Human Immunodeficiency Virus (HIV) infected cells (lane 1) but not in non-infected cells (lane 2). Lane 3 is a mycoplasma positive control and lane 4 shows DNA size markers. The arrow is pointing to the mycoplasma product (270bp). The cDNA fragments were obtained by RT-PCR experiments;
**Figure 3** is a pie chart showing the distribution of organisms when running the deep sequencing output against all human transcripts combined with all bacterial transcripts;
**Figure 4A** is a pie chart showing the distribution of human RNA transcripts in the uninfected samples. The chart includes only transcripts with a base mean (normalized read count mean for each sample (condition)) of more than 20;
**Figure 4B** is a pie chart showing the distribution of human RNA transcripts in the infected samples. The chart includes only transcripts with a base mean of more than 20; and
**Figure 5** is a plot showing ncRNA transcript expression in the uninfected vs. the infected samples (Base mean1 and Base mean2, respectively).

### DETAILED DESCRIPTION OF THE INVENTION

Described herein is a method for the detection of a microorganism infection using short reads, generated by deep sequencing of short RNA extracts. The method comprises one or more of the following steps: (i) isolating a fraction of small RNA molecules from a biological sample of the subject; (ii) alignment of the short reads against the subjects' reference genome; (iii) subtraction and assembly of the remaining unmapped reads; and (iv) categorization and identification of infection by the microorganism by using nucleic acid databases and/or computational alignment tools. The method is advantageous over currently known methods in that it identifies minute quantities of non-host microorganisms. Such improved sensitivity is provided by the use of small RNA molecules rather than large RNA molecules or cDNA molecules which enhances the microorganism to host ratio. The method is useful for an early and accurate detection of microorganism(s) in clinical and environmental samples.

### Definitions

To facilitate an understanding of the present invention, a number of terms and phrases are defined below. It is to be understood that these terms and phrases are for the purpose of description and not of limitation, such that the terminology or phraseology of the present specification is to be interpreted by the skilled artisan in light of the teachings and guidance presented herein, in combination with the knowledge of one of ordinary skill in the art.

As referred to herein, the terms "nucleic acid", "nucleic acid molecules" "oligonucleotide", "polynucleotide", and "nucleotides" may interchangeably be used. The terms are directed to polymers of deoxyribonucleotides (DNA), ribonucleotides (RNA), and modified forms thereof in the form of a separate fragment or as a component of a larger construct, linear or branched, single stranded, double stranded, triple stranded, or hybrids thereof. The term also encompasses RNA/DNA hybrids. The polynucleotides may include sense and antisense oligonucleotide or polynucleotide sequences of DNA or RNA. The DNA molecules may be, for example, but not limited to: complementary DNA (cDNA), genomic DNA, synthesized DNA, recombinant DNA, or a hybrid thereof. The RNA molecules may be, for example, but not limited to: mRNA, tRNA, shRNA, siRNA, miRNA, snRNA, snoRNA, rRNA and the like. The terms further include oligonucleotides composed of naturally occurring bases, sugars, and covalent internucleoside linkages, as well as oligonucleotides having non-naturally occurring portions, which function similarly to respective naturally occurring portions.

The term "sequencing" is directed to a process for determining the nucleotide sequence of a polynucleotide molecule. The term "read(s)" is directed to the output of the sequencing process. In some embodiments, the read is the sequence of a nucleic acid. The term "short read(s)" is directed to a read which is derived from sequencing of a short RNA polynucleotide. In some embodiments a short read may include a sequence of 10-100 nucleotides. In some embodiments a short read may include a sequence of 16-50 nucleotides. In some embodiments a short read may include a sequence of 20-30 nucleotides

The term "filter" as used herein is directed to the elimination or subtraction of records (such as sequences) from a database.

The term "unmapped reads" is directed to reads which do not align/match aligning/comparing a read against a reference database. The term "mapped reads" is directed to reads which align/match to a sequence in a reference database.

The terms "small RNA", "small RNA molecule(s)" "short RNA" and "short RNA polynucleotide(s)" may interchangeably be used and are meant to include all type of small/short RNA molecules, which have a length of about 10-100 nucleotides. In some embodiments, the small RNA molecules have a length in the range of about 16-50 nucleotides. In some embodiments, the small RNA molecules have a length in the range of about 20-30 nucleotides. In some embodiments, the small RNA molecules may be coding RNA molecules, or fragments thereof. In some embodiment, the small RNA molecules may be non-coding RNA molecules (ncRNAs), or fragments thereof. In some embodiments, the ncRNA molecules play crucial roles in cellular functions including, but not limited to: modulation of the biogenesis and activity of ribosomes, repression of gene expression, facilitation of mRNA splicing, regulation of transcription factors, alteration of cellular proliferation and apoptosis, and participation in infrastructural functions. In some embodiments, the small RNA molecules comprise such molecules as: small interfering RNA (siRNA), micro RNA (miRNA), small heterochromatic RNA (shRNA), Piwi-interacting RNA (piRNA), small nuclear RNA (snRNA), small nucleolar RNA (snoRNA), and/or fragments of larger RNA molecules, such as, for example, transfer RNA (tRNA), ribosomal RNA (rRNA) and the like. In further embodiments, the small RNA molecules may include any RNA molecule at the length of about 1-100 nucleotides that may be a degradation product of a larger RNA molecule (such as, for example, mRNA), or any other RNA molecule in the length of about 1-100 nucleotides. In some embodiments, the small RNA may be a single stranded RNA. In other embodiments, the small RNA may be a double stranded RNA. Small RNAs may be formed by various mechanisms. In some cases, small RNAs are derived from dsRNA precursors in cells that may act as guide RNAs during sequence-specific epigenetic regulation of eukaryotic gene expression. In some cases, small RNAs may form as a result of specific cleavage of RNA molecules in the cells, wherein the RNA molecules may be derived from a microorganism infecting the cells. In some cases, the small RNAs may form as a degradation process of larger RNA molecules. Procedures to obtain and or record the profile of small RNAs expressed in cells or tissues are described, for example, in a publication by Sébastien Pfeffer et.al. 2005.

The term "contig" is directed to a set of overlapping DNA or RNA segments derived from a single genetic source. A contig can be used to deduce the original DNA or RNA sequence of the source. In some embodiments, a contig is an RNA sequence reconstructed (assembled) from a set of overlapping small RNA sequences. In some embodiments, RNA sequence assembly can be performed by any algorithm or software program. In some embodiments, the RNA sequence assembly is performed using velvet's assembly assembler. In some embodiments, the contig may have a length of about 10-500 nucleotides. In some embodiments, the contig may have a length of about 50-150 nucleotides. In some embodiments, the contig may have a length of about 50-100 nucleotides. In some embodiments, the contig may have a length of about 70-100 nucleotides. In some embodiments, the contig may have a length of 75 nucleotides. In some embodiments, the contig may have a length of 91 nucleotides. In some embodiments, the better the assembly is, the longer the contigs are.

The terms "alignment" and "aligning" are directed to the process of comparing/matching of a first nucleotide sequence to one or more additional nucleotide sequences. Alignment may be performed by any of the methods known in the art, such as, for example, by use of computational analysis softwares. In some embodiments, the computational analysis software is any available sequence analysis program (commercially or non commercially). In one embodiment, the alignment is performed using the read and analyze (RandA) software. In another embodiment, the alignment is performed using the Burrows-Wheeler Aligner (BWA) alignment software. In another embodiment, the alignment is performed using the TopHat alignment software. In another embodiment, alignments is performed by alignment program tools including, but not limited to: deep sequencing small RNA analysis pipeline (DSAP), miRTools, miRExpress, miRNAkey, ncRNA read and analyze (RandA), BWA alignment software, Stampy, Basic Local Alignment Search Tool (BLAST), BLAT, Novoalign, Bowtie, Mosaik, Blat-like Fast Accurate Search Tool (BFAST), Micro Read Fast Alignments Search Toll (mrFAST), Mapping and Assembly with Quality (MAQ), LALIGN,, BLASTN, TopHat, BWA alignment software, and the like) and it may be adjusted by various parameters, such as, for example: degree of similarity/identity between the compared nucleotide sequences; length of the compared nucleotide sequences, quality of read (sequencing). "Optimal alignment" is defined as an alignment giving the highest percent identity score. In some embodiments, the first nucleotide sequence is a nucleotide sequence derived from a small RNA molecule sequence and the alignment is performed between the first nucleotide sequence and a one or more additional sequences, the sequence of which is found in a database.

As referred to herein, the term "database" is directed to an organized collection of nucleotide sequence information that may be stored in a digital form. In some embodiments, the database may include any sequence information. In some embodiments, the database may include the genome sequence of a subject or a microorganism. In some embodiments, the database may include expressed sequence information, such as, for example, an EST (expressed sequence tag) or cDNA (complementary DNA) databases. In some embodiments, the database may include non-coding sequences (that is, untranslated sequences), such as, for example, the collection of RNA families (Rfam) which contains information about non-coding RNA genes, structured cis-regulatory elements and self-splicing RNAs. In exemplary embodiments, the databases may be selected from redundant or non-redundant GenBank databases (which are the NIH genetic sequence database, an annotated collection of all publicly available DNA sequences). Exemplary databases may be selected from, but not limited to: GenBank CDS (Coding sequences database), PDB (protein database), SwissProt database, PIR (Protein Information Resource) database, PRF (protein sequence) database, EMBL Nucleotide Sequence database, and the like, or any combination thereof.

The terms "deep sequencing" and "next generation sequencing" may interchangeably be used and are directed to an enhanced sequencing method enabling the rapid parallel sequencing of multiple nucleic acid sequences. In some embodiments, deep sequencing is used to infer transcript expression levels. In some embodiments, deep sequencing is directed to sensitive and specific method which identifies transcripts with low expression level. In some embodiments, deep sequencing allows the identification of novel ncRNA transcripts. In some embodiment, deep sequencing does not require any prior knowledge on the transcripts' sequence.

The term "subject" is directed to a host organism capable of harboring a microorganism from different specie(s). In some embodiments, the subject is a mammal, such as a human. In some embodiments, the subject is an animal which is not a mammal (such as, for example, avian, fish, and the like). In some embodiments, the subject is a plant.

The term "microorganism", as used herein is directed to an organism that can reside (habitat/infect/live) within the subject body and/or within a cell of the subject. The microorganisms may include such microorganisms as, but not limited to: virus, bacteria, fungi, parasite, amoeba and the like. In some embodiments, the microorganism is a pathogen capable of causing a disease and/or clinically detectable symptoms in the subject. In some embodiments, infection by the microorganism may not cause a clinically detectable symptom. In some embodiments the microorganism is a symbiotic microorganism. In some embodiments, the microorganism may reside within the subject body, on the subject body (such as, for example, on the subject's skin) and/or within one or more cells of the subject. In some embodiments, all or part of the microorganism genome may be integrated into the genome of the subject. In some embodiments, neither the entire genome nor parts of the genome of the microorganism integrate into the genome of the subject. In some embodiments the microorganism is a virus, such as, for example, dsDNA viruses (such as, for example, Adenoviruses, Herpesviruses, Poxviruses), ssDNA viruses (such as, for example, Parvoviruses), dsRNA viruses (such as, for example, Reoviruses), (+)ssRNA viruses (+)sense RNA (such as, for example, Picornaviruses, Togaviruses), (-)ssRNA viruses (-)sense RNA (such as, for example, Orthomyxoviruses, Rhabdoviruses), ssRNA-RT viruses (+)sense RNA with DNA intermediate in life-cycle (such as, for example, Retroviruses), dsDNA-RT viruses (such as, for example, Hepadnaviruses). In some embodiments, the microorganism is a bacteria, such as, for example, a gram negative bacteria, a gram positive bacteria, and the like. In some embodiments, the microorganism is a fungi, such as yeast, mold, and the like. In some embodiments, the microorganism is a parasite, such as, for example, protozoa and helminths or the like. In additional embodiments, the microorganism may be selected from archaea, protists; microscopic plants (green algae), plankton, and the planarian. In some embodiments, the microorganism is unicellular (single-celled). In some embodiments, the microorganism is multicellular. In some embodiments, the microorganism may cause cancer. In some embodiments, the microorganism may cause Acquired Immune Deficiency Syndrome (AIDS). In some embodiments, the microorganism may cause infectious mononucleosis.

The term "infecting", as used herein is directed to the presence of a microorganism within a subject body and/or a subject cell. For example, a virus may be infecting a subject cell. A parasite (such as, for example, a nematode) may be infecting a subject cell/body.

The term "construct", as used herein, refers to an artificially assembled or isolated nucleic acid molecule which may include one or more nucleic acid sequences, wherein the nucleic acid sequences may include coding sequences (that is, sequence which encodes an end product), regulatory sequences, non-coding sequences, or any combination thereof. The term construct includes, for example, vector but should not be seen as being limited thereto.

The sequenced nucleic acids may be introduced or transfected or into target cells. As used herein, the terms "introducing" and "transfection" may interchangeably be used and refer to the transfer of molecules, such as, for example, nucleic acids, polynucleotide molecules, vectors, and the like into a target cell(s), and more specifically into the interior of a membrane-enclosed space of a target cell(s). The molecules can be "introduced" into the target cell(s) by any means known to those of skill in the art, for example as taught by Sambrook et al. Molecular Cloning: A Laboratory Manual, Cold Spring Harbor Laboratory Press, New York (2001), the contents of which are incorporated by reference herein. Means of "introducing" molecules into a cell include, for example, but are not limited to: heat shock, calcium phosphate transfection, PEI transfection, electroporation, lipofection, transfection reagent(s), viral-mediated transfer, and the like, or combinations thereof. The transfection of the cell may be performed on any type of cell, of any origin, such as, for example, human cells, animal cells, plant cells, virus cell, and the like. The cells may be selected from isolated cells, tissue cultured cells, cell lines, cells present within an organism body, and the like.

The terms "polypeptide," "peptide" and "protein" are used interchangeably herein to refer to a polymer of amino acid residues. The terms apply to amino acid polymers in which one or more amino acid residue is an artificial chemical analogue of a corresponding naturally occurring amino acid, as well as to naturally occurring amino acid polymers.

According to some embodiments, there is provided a method that allows the rapid, accurate and sensitive detection of infection of a subject by a microorganism. Further, since the method is unbiased towards a specific microorganism it may provide an added level of confidence as it may be used for the detection of all known microorganisms that may infect a given subject. Additionally, the method may be quantitative alternatively or in addition to being qualitative.

According to some embodiments, there is thus provided a method for the detection and identification of an infection of a subject by a microorganism. The method comprising the steps of (i) alignment of short reads derived from small RNA molecules, against the subject reference genome database; (ii) filtering (subtraction) and assembly of the remaining unmapped reads to produce longer contigs; and (iii) categorization and identification of the infection by a microorganism based on nucleic acid databases.

The term "differential expression analysis" as used herein refers to the analysis of differentially expressed reads of RNA transcripts in each sample. In some embodiments, the analysis comprises calculation of transcript expression variance. In some embodiment, calculation of transcript expression variance allows an accurate and powerful assessment of the difference in expression between different conditions (samples). In some embodiments, the differential expression analysis is performed using the "R"-based tool, DESeq. In some embodiments, the DESeq utilizes a negative binomial distribution model for variance estimation.

In some embodiments, the subject is a mammal, such as a human. In some embodiments, the subject is a mammal animal. In some embodiments, the subject is a non-mammal animal, such as, for example, an avian, a fish, and the like. In some embodiments, the subject is a plant.

In some embodiments, the microorganism may comprise a virus, a bacteria, a fungi, a parasite, or combinations thereof. According to some embodiments the microorganism is a virus, such as, for example, dsDNA viruses (such as, for example, Adenoviruses, Herpesviruses, Poxviruses), ssDNA viruses (such as, for example, Parvoviruses), dsRNA viruses (such as, for example, Reoviruses), (+)ssRNA viruses (+)sense RNA (such as, for example, Picornaviruses, Togaviruses),(-)ssRNA viruses (-)sense RNA (such as, for example, Orthomyxoviruses, Rhabdoviruses), ssRNA-RT viruses (+)sense RNA with DNA intermediate in life-cycle (such as, for example, Retroviruses), dsDNA-RT viruses (such as, for example, Hepadnaviruses). In some embodiments, the microorganism is a bacteria, such as, for example, a gram negative bacteria, a gram positive bacteria, and the like. In some embodiments, the microorganism is a fungi, such as yeast, mold, and the like. In some embodiments, the microorganism is a parasite, such as, for example, protozoa and helminths or the like. In some embodiments, the infection by the microorganism may inflict a disease and/or a clinically detectable symptom to the subject. In some embodiments, infection by the microorganism may not cause a clinically detectable symptom. In some embodiments, the microorganism is a symbiotic microorganism. In additional embodiments, the microorganism may comprise archaea, protists; microscopic plants (green algae), plankton, and the planarian. In some embodiments, the microorganism is unicellular (single-celled). In some embodiments, the microorganism is multicellular.

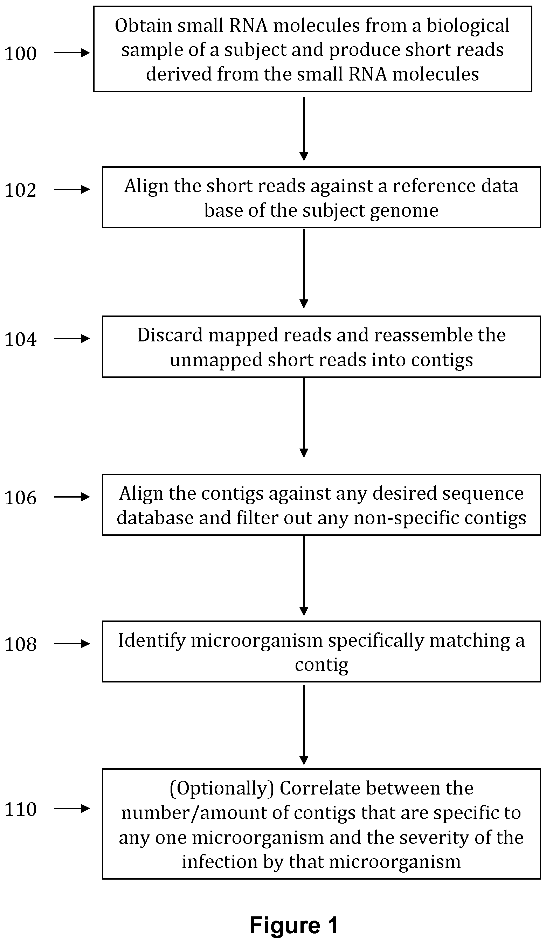

Reference is now made to Figure 1, which illustrates a flow chart of steps of the method for the detection and identification of infection of a subject by a microorganism, according to some embodiments. In step 100, short sequences (short reads) derived from short (small) RNA molecules obtained from a biological sample of the subject are produced. Next, in step 102, the short reads are aligned against a reference database of the subject genome. In step 104, unmapped reads (that is, small RNA derived sequences that did not align with the subject genome) are reassembled (by de-novo synthesis process) to produce longer contigs (of for example, about 50-500 nucleotides in length). Next, in step 106, the contigs are aligned (matched) against any desired sequence database (such as, for example, any known organism databases) and non specific contigs (that is, contigs which are not specific to one or more organism/microorganism) are filtered out (discarded). In step 108, the organism/microorganism (single or multiple) uniquely/specifically matching a contig sequences are identified and determined.

In optional step 110, the relative amount of contigs which match only one specific microorganism is correlated to the degree/severity of infection of the microorganism.

In some embodiments, in steps 106 and 108, in order to improve the accuracy and sensitivity of the method, ranking of the results may be performed. For example, only the highest ranking alignments (having the lowest E-value and highest total score) may be included in the analysis. Using the alignment results (as performed, for example, by BLAST software), a microorganism table may be prepared for each tested sample. In order to determine a unique hit (that is, a matched alignment to a contig, that is determined to be specific to only one microorganism), and to add the suspected microorganism to the table, only the highest ranking alignments, which matched only a single microorganism are used. In other exemplary embodiments, the multiple alignment (BLAST) results could be categorized using a common microorganism for all the hits in a query (for example if a sequence aligns to two different viral agents then the method will count it as one unique hit for "Viruses" composed of 2 different viruses). Additionally, multiple unique hits may be counted for each unique microorganism that matched a contig. The microorganism's total E-Value may further be calculated by multiplying the E-Value of all of its unique hits. Additionally, to account for multiple hits for a specific microorganism, a microorganism total score (also referred to herein as "confidence level") may be calculated by summing the score for each unique hit with the score for each non-unique hits obtained for that microorganism, and divided by the number of various microorganisms identified per each query (round). The confidence score may be indicative of the confidence level that the microorganism is indeed infecting the subject. In some embodiments, a standard of maximum total E-value may be determined and used as a cut-off limit to exclude hits as being non-unique. In some exemplary embodiments, a maximum total E-value of 1*E⁻²⁰⁰ may be used.

In some embodiments, in steps 106 and 108, a differential expression analysis of the deep sequencing data is performed. The differential expression analysis is described by the base mean value which is calculated using the DESeq tool. Base Mean represents the mean normalized read count for each condition (sample). The Base mean is calculated using the DESeq tool which calculates a size factor for each condition by first constructing a "reference sample" by calculating for each gene, the geometric mean of the counts in all samples. Then, the quotient of the counts in the sample divided by the counts of the reference sample is calculated for each gene. Finally, the median of all the quotients are calculated to get a relative depth of the sample. This depth is used to normalize the read count for each gene in a sample. After normalization, a mean read count is calculated for each gene, for each condition.

According to some embodiments, and as further detailed below, determining the microorganism confidence score may further be used to quantitate the degree/severity of the infection.

According to some embodiments, the step of assembly (for example, step 104 in Fig. 1) provides further advantages to the method and enables the identification of microorganism infection with much higher probability, sensitivity and accuracy. In some embodiments, the step of assembly of the short sequences derived from the small RNA molecules to larger contigs, vastly increases the sensitivity of the method and enables the accurate identification of microorganisms, that otherwise would not have been identified.

In some embodiments, there is provided a method (also designated, in some embodiments, as ncRNA read and analyze (RandA)) for analyzing sequences. The method may be further used in methods for detecting of infection of a subject by a microorganism. In some embodiments, the method comprises: (i) analyze an input of deep sequencing data derived from a fraction of small RNA molecules from a biological sample of a subject; (ii) align the nucleic acid sequences against a database comprising the subject nucleic acid genome sequence; (iii) generate a new database of the aligned transcripts and collapse the database by reducing identical redundant sequences; (iv) perform differential expression analysis of the uniquely and ambiguous mapped sequences; and (v) identify a microorganism based on the output of the differential expression analysis. In some embodiments, the small RNA molecules are non coding RNA molecules. In some embodiments, the database is Rfam database. In some embodiments, the expression analysis is performed on uniquely and multiple mapped reads. In some embodiments, the multiple mapped reads are analyzed using a SEQ-EM algorithm. In some embodiments, the differential expression analysis further comprises statistical analysis of the deep sequencing data obtained from one sample or a plurality of replicate samples assigned under a specific condition. In some embodiments, the statistical analysis of one sample is performed using a chi-squared test. In some embodiments, the chi-squared test is performed to obtain P-values under the null hypotheses of no differential expression between samples and corrected according to the Bonferroni correction for multiple hypotheses testing. In some embodiments, the differential expression analysis of plurality of replicate samples is performed using a DESeq tool. In some embodiments, the differential expression analysis is described by the base mean value. In some embodiments, the base mean value represents the mean normalized read count for each condition (sample). The base mean is calculated using the DESeq tool which calculates a size factor for each condition by first constructing a "reference sample" by calculating for each gene, the geometric mean of the counts in all samples. Then, the quotient of the counts in the sample divided by the counts of the reference sample is calculated for each gene. Finally, the median of all the quotients are calculated to get a relative depth of the sample. This depth is used to normalize the read count for each gene in a sample. After normalization, a mean read count is calculated for each gene, for each condition.

According to some embodiments, and without wishing to be bound to theory or mechanism, the use of small RNA molecules in the method for the detection and identification of infection of a subject by a microorganism results in a very sensitive, unbiased and accurate method (as further exemplified hereinbelow). This may be attributed, at least in part to the high ratio of nucleotide sequences derived from the microorganism to the nucleotide sequences derived from the subject. Additionally, the preparation and use of small RNA molecules may exclude the highly abundant RNA species (such as, for example, rRNA and tRNA) that are of subject origin, that may provide a very high background and obscure small RNA derived sequences.

According to additional embodiments, further advantage of the method that may be attributed to the use of small RNA molecules is that the method may be used as a quantitative method in addition to or alternatively to being qualitative. Accordingly, in some embodiments, the method of detection may be qualitative. By qualitative it is meant that the method may be used to detect the presence of a microorganism, (that is, provide a yes/no answer as to the presence of a specific microorganism within the biological sample). In some embodiments, the method of detection may be quantitative. By quantitative it is meant that the method may be used to quantitate the amount and/or degree of the infection by the microorganism of the subject, and hence provide an estimate for the severity of that infection. For example, a result indicating the presence of high copy of microorganism presence may be used to estimate the severity of the infection, the time that has passed since the infection, and the like. In some embodiments, the total score of the specific microorganism may be used for the quantitation of the severity of the infection. Such a quantitative method may be used as a valuable tool in determining a treatment regime for the specific infection and the severity of that infection.

In some embodiments, the methods can be implemented for various uses, such as, for example, clinical use for detecting and/or identifying of an infection of a subject by a microorganism. In some embodiments, the method can be implemented for identification of HIV 1 infection. In some embodiments, the method can be implemented for identification of CMV 5. In some embodiments, the method can be implemented for identification of HPV 18.

According to some embodiments, the small RNA molecules may be obtained from a biological sample of the subject. The biological sample may be any appropriate body-derived sample. For example, the biological sample may be selected from, but not limited to: fluid samples such as whole blood, peripheral blood monocytes, leukocytes; various cells and tissues; fixed and/or embedded tissue sections; the biological sample may be either freshly extracted or frozen. In some embodiments, the biological sample may be obtained from a living or dead subjects. Obtaining the small RNA molecules may be performed by any method known in the art (such as disclosed, for example, in the "Small RNA v1.5 Sample preparation Guide, Illumina, Sept. 2010 version). In some embodiments, after obtaining the small RNA molecules from the biological sample, the small RNA molecules may be further processed so as to adjust their use in the method. For example, the small RNA molecules may be ligated to a 3' adapter that may be specifically modified to target the small RNA molecules, which have a 3' hydroxyl group (resulting from its enzymatic processing). The 3' adapter may be used for the step of reverse transcribing the small RNA molecules. The small RNA molecules may be further ligated to a 5' adapter which is used for amplification of the small RNA segments.

In some embodiments, one or more steps in the method may be performed using one or more computational system. The computation system may include hardware and/or software that may be utilized in the method. Exemplary software known in the art that may be used in the method include, but is not limited to: Read-and-Analyze (RandA) software, BLAST, LALIGN, FASTA, FASTX, BWA alignment software, Velvet assembly software, Top Hat, NCBI's E-utils (http://eutils.ncbi.nlm.nih.gov) or any other database resources, alignment tools, clipper tools, assembler tools or other analysis tools either known today or to be developed in the future. In some embodiments, the analysis tools may be commercially available. In some embodiments the analysis tools may be specifically developed.

According to further embodiments, there is provided a system for detecting infection of a subject by a microorganism, the system comprising: a sequencing device configured to determine nucleic acid sequences derived from small RNA molecules obtained from a biological sample of a subject; and a computing unit configured to: i) align the nucleic acid sequences against a database comprising the subject nucleic acid genome sequence; ii) subtract and assemble unmapped sequences to produce contigs; and iii) identify a microorganism based on alignment of the contigs against databases comprising nucleic acid sequences. In some embodiments, the computing unit is further configured to calculate a confidence score indicative of the confidence level that a microorganism is infecting the subject. In some embodiments, the sequencing device is a deep sequencer. In some embodiments, the computing unit may be selected from any suitable computational device or computational system.

In some embodiments, the computational unit/device/system may incorporate at least a central processing unit (CPU), a temporary memory such as Random Access Memory (RAM) and a computer-readable medium or article such as a non-transient memory. Non-transient memory stores a set of instructions that, when executed by CPU, cause the CPU to perform at least some of the steps in the method of detecting of infection of a subject by a microorganism and/or other operations in accordance with present embodiments. The computer-readable medium or article may include, for example, any type of disk including a floppy disk, an optical disk, CD-ROM, a read-only memory (ROM), a random access memory (RAM), flash memory, an electrically programmable read-only memory (EPROM), an electrically erasable and programmable read only memory (EEPROM), or any other type of media suitable for storing computer instructions, and capable of being coupled to a computer system bus. The instructions may include any suitable type of code, for example, source code, compiled code, interpreted code, executable code, static code, dynamic code, or the like, and may be implemented using any suitable high-level, low-level, object-oriented, visual, compiled and/or interpreted programming language, such as C, C++, C#, Java, BASIC, Pascal, Fortran, Cobol, assembly language, machine code, and/or the like.

In some embodiments, there is provided a non-transitory computer-readable medium having stored therein instructions for detecting of infection of a subject by a microorganism, wherein the instructions, when executed by the computer, cause the computer to: a) align nucleic acid sequences derived from small RNA molecules obtained from a biological sample of a subject, against a database comprising the subject nucleic acid genome sequence; b) subtract and assemble unmapped sequences to produce contigs; and c) identify a microorganism based on alignment of the contigs against databases comprising nucleic acid sequences.

The following examples are presented in order to more fully illustrate certain embodiments of the invention. They should in no way, however, be construed as limiting the broad scope of the invention.

### EXAMPLES

### Example 1: Detection/identification of microorganism infection of subject cells: Detection/identification of Human Immunodeficiency Virus (HIV) and mycoplasma infection of human cells.

The method for microorganism detection using small RNA extracts is hereby applied on cells infected with HIV. In some instances, the method may be termed herein "short RNA subtraction and assembly (SRSA)".

### Infection of SupT1 cells with HIV-1, and RNA sample preparation:

SupT1 cells (human Caucasian pleural effusion lymphoma, T cells) were infected with HIV-1 (HXB2 strain) on day 0. Four days post infection, about 50% of naive cells (control) were added to the cultures and four days later the cells were harvested. Total RNA was extracted from the cells using TRIzol reagent (Invitrogen™). 10 µg of each RNA sample were prepared for Deep-sequencing following the manufacturer small RNA sample preparation protocol v1 (Illumina®). Briefly, samples were run on gel and fragments of 10-50 nt were isolated from the gel. Then, samples were ligated with 3' and 5' adapters, reverse-transcribed and PCR amplified. cDNA libraries were prepared from PCR products of about 93-100 nt (representing about 20-30 nt RNA products) and sequenced in separate lanes on an Illumina Genome Analyzer IIx instrument (Illumina®).

### Alignment and assembly:

The reads were clipped for the standard short RNA adapter using fastx clipper, discarding all reads shorter than 16 nt. The sequencing method produced 21,048,677 and 12,003,830 reads after clipping of the control (HIV negative) and test (HIV positive) samples, respectively. The reads were then aligned using Burrows-Wheeler Aligner (BWA) alignment software (default parameters) against the human genome (hg19) reference retrieved from NCBI. Samples were re-aligned using TopHat, which considers splice junctions in the alignment process, to test the confounding effect of splice junctions presence on the results. Since BWA demonstrated higher mapping accuracy (83% as opposed to 78% using TopHat), all downstream analysis was conducted on its output. Filtering the human genome-associated reads produced 1,251,267 (6%) and 1,992,557 (16.6%) unmapped reads in the HIV-1 negative and positive samples, respectively.

In order to reduce the number of multiple organism hits yielded from matching such short reads against a large database, read groups were assembled into longer contigs. The unmapped reads were assembled, using Velvet's Assembly Assembler (v1.3), which conducts assemblies with predefined parameter values across a user-specified range of k-mer values, followed by utilization of the contigs from all previous assemblies, as input for a final assembly. The script was then ran with a wide range of hash lengths of 9-31 nt in order to optimize the length of the contigs. In some cases, the most effective length was found to be within the range of about 17 to 25 nt. The assembly produced 16 and 878 contigs with an average length of 75 and 91 nt for the HIV-1 negative and positive samples, respectively. The difference in the amount of produced reads could be accounted for by the lack of non-human-associated sequences in the negative (control, non infected) sample.

### Categorization and identification:

To match the assembled contigs against any known organism , the contigs were aligned using NCBI's nucleotide megablast with a word size of 28, against the "All non-redundant GenBank CDS translations+PDB+SwissProt+PIR+PRF" database, with an inclusion threshold *E*-value of 0.01. Using an in-house developed software, only the highest ranking hits (with lowest E-value and highest total score) were included per query for the downstream analysis. Using the BLAST results, a microorganism table was prepared for each sample (Table 1 herein). Unique microorganism hits were added when all the highest ranking hits per query matched only a single organism (organism taxonomy retrieved using NCBI's E-utils (http://eutils.ncbi.nlm.nih.gov/) and taxonomy database). Multiple hits were counted for each organism matched per query. The organism's total E-value was calculated by multiplying the E-value of all the unique hits (E-value is nearly identical to P-values for E<0.01). To account for the multiple hits in the blast results, an organism total score were calculated, by summing the score for each unique organism hit with the score for each multiple hit, divided by the number of different organisms per query. This summation approach did not increase the interspecies differentiation capabilities, since different species could have a different number of strains and annotations. It was, however, utilized for intraspecies analysis, prioritizing the different strains (Table 2 herein). Deciding on a standard of maximum total E-value of 1*E⁻²⁰⁰, no organism was identified in the HIV-1 negative sample, the most prominent was Homo sapiens with nine unique hits. In the HIV-1 positive sample, four organisms were identified, one of which was Homo sapiens, the other three were Mycoplasma Hyorhinis HUB-1, Human Immunodeficiency Virus 1 and Mycoplasma Hyorhinis (Table 1 herein).

Using the method, both infecting microorganism and host-related sequences were detected with very high certainty and an extremely low E = 0. Such high accuracy of detection is indicative of high sensitivity of the method. It was also observed that the number of different taxonomies passing the basic filter of having the highest query score and lowest E-value was very high, demonstrating a low specificity rate. Out of 385 different taxonomies passing the basic filter, 14 were Mycoplasma out of which 3 were of the Hyorhinis strain. There were also 19 different human immunodeficiency virus taxonomies and 1 Homo sapiens. Counting only the Hyorhinis, HIV-1 and Homo sapiens as true positives (true positive rate; TPR = 0.06), the method demonstrated a false positive rate (FPR) of 0.94. A more rigorous inclusion criteria can be implemented to reduce the FPR, while maintaining high sensitivity. Setting the inclusion criteria to only include taxonomies that have unique query hits, resulted in eight different taxonomies, one of which is Homo sapiens, one HIV-1 and four Mycoplasma out of which two are Hyorhinis reaching an FPR of 0.5. A maximal E-value threshold equal to 1*E⁻²⁰⁰ was then added. Such restriction resulted in only four taxonomies remaining, all of which are true positives, with Mycoplasma Arginini being the closest taxonomy for inclusion with 4*E⁻¹⁸¹. The total score, calculated by dividing each query score against the number of taxonomies it hits and summing it for each taxonomy, could also serve as a reliable filtration standard, though further tests are required.

**Table 1. Pathogens identified in the samples using the method of the invention.**

| **Organism** | **Unique Hits** | **Multiple Hits** | **Total E-value** | **Total Score** |
|---|---|---|---|---|
| Mycoplasma hyorhinis HUB-1 | 564 | 51 | 0 | 48951 |
| Mycoplasma hyorhinis | 12 | 42 | 0 | 1544 |
| Human immunodeficiency virus 1 (HIV-1) | 13 | 26 | 0 | 1138 |

**Table 2. Distribution of HIV related BLAST hits.**

| **HIV hit name** | **Accession No.** | **Total hits** |
|---|---|---|
| HIVHXB2CG HIV type 1 (HXB2), complete genome; HIV1/HTLV-III/LAV reference genome \| 1906382 | K03455/ M38432 | 1.147 |
| HIV-1, complete genome \| 9629357 | NC_001802 | 0.809 |
| Human T-cell leukemia type III (HTLV-III) proviral genome \| 61569 | X01762 | 0.475 |
| HIVBH102 HIV type 1, isolate BH10, genome \| 326383 | M15654 | 0.475 |
| HIVPV22 HIV type 1, isolate PV22, complete genome (H9/HTLV-III proviral DNA) \| 555008 | K02083 | 0.454 |
| HIVTH475A HIV type 1 (individual isolate: TH4-7-5) gene \| 474287 | L31963 | 0.333 |
| HIV-1 isolate F233 from Argentina vpu gene, complete sequence \| 122912310 | EF119921 | 0.333 |
| HIVH3BH5 HIV type 1, isolate BH5 \| 327459 | K02012 | 0.316 |
| HIV-1 proviral vif gene DNA \| 1127949 | U42272 | 0.294 |
| HIVMCK1 HIV 1 DNA \| 1398973 | D86068 | 0.275 |

### Example 2 -Identification of the presence of infection by a microorganism by RT-PCR experiments.

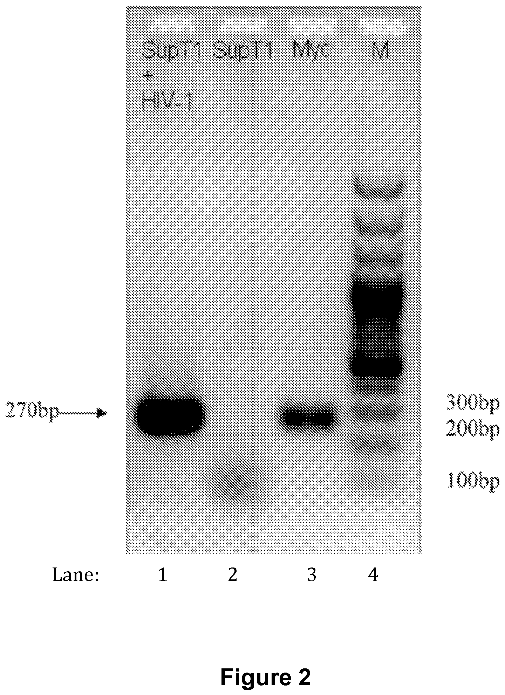

To confirm the detection of Mycoplasma contamination in the HIV-infected cells (as determined in Example 1), an RT-PCR experiment was performed on the SupT1 cells. RNA samples from the cells (50 ng and 200 ng in 50 µL reaction volume) were reverse transcribed using High-Capacity Reverse Transcription Kit with random primers (Applied Biosystems™) (1jug RNA in 15µL total reaction volume) and used in the EZ-PCR Mycoplasma test kit (Biological Industries, Beit-Ha'Emek). The results are presented in Figure 2, which shows a picture of PCR products in 1.5% Agarose gel. As can be seen, in lane 1 (HIV infected cells), a single PCR band which correspond to mycoplasma (270 bp, arrow) can be identified, whereas in lane 2 (control, non-infected cells), no presence of mycoplasma is observed. Lane 3 is a positive control for mycoplasma. Lane 4 represents markers. Thus, the results presented in Example 2 confirm the fidelity, sensitivity and the unbiased method for the detection of infection of a subject by a microorganism by utilizing small RNA derived sequences.

### Example 3: Detection/identification of microorganism infection of subject cells: Detection/identification of a cytomegalovirus (CMV) and HIV infection of human cells.

### Infection of SupT1 cells with CMV, and HIV and RNA sample preparation:

Total RNA was extracted from HIV-1 infected SupT1 cells and from CMV infected primary fibroblasts. cDNA libraries of the samples were prepared as described in example 1. Equal amounts of the cDNA libraries were pooled and then sequenced using the Illumina platform "MiSeq", according to manufacturer instructions/protocols.
The resulting sequencing obtained a total of 7,157,364 reads.

### Alignment and assembly:

The sequenced reads were clipped for the standard short RNA adapter using fastq-mcf clipper. Discarding 616,429 reads which are shorter than 16 nt and filtering 465,718 reads on purity flag resulted in 6,075,217 reads.

### Detection of CMV infection of human cells:

The reads were then aligned using Burrows-Wheeler Aligner (BWA) alignment software (default parameters) against the human genome (hg19/GRCh37) reference downloaded from UCSC index 10 (CMV). The alignment resulted with 2,452,378 mapped reads (80.14%). Filtering the human genome-associated reads produced 607,726 (19.86%) unmapped reads. Aligning of the unmapped reads against the CMV-AD169 reference displayed a very high presence of 566,280 mapped reads of CMV reads (93.18%).

### Detection of HIV infection of human cells:

Alignment against the human genome (hg19/GRCh37) reference downloaded from UCSC index 11 (HIV) resulted with 2,815,500 mapped reads (93.38%). Filtering the human genome-associated reads produced 199,613 (6.62%) unmapped reads. Aligning of the unmapped reads against the HIV reference displayed 13,071 mapped reads (6.548%).When utilizing SRSA on all the unmapped reads from both CMV and HIV indexes, 135 contigs in which the average read length was 113 nt (the maximal read length was 399 and the minimal read length was 54) were obtained.

### Categorization and identification:

The contigs were then aligned using NCBI's nucleotide megablast with a word size of 28, against the "All non-redundant GenBank CDS translations+PDB+SwissProt+PIR+PRF" database, with an inclusion threshold *E*-value of 0.01. Using an in-house developed software, only the highest ranking hits (with lowest E-value and highest total score) were included per query for the downstream analysis. Using the BLAST results, a microorganism table was prepared, for the analysis of the HIV infection detection (Table 3) and the analysis of the CMV infection detection (Table 4).

**Table 3: Detection of HIV 1 infection.**

| **Organism** | **Unique organism hits** |
|---|---|
| Human herpesvirus 5 | 75 |
| HIV 1 | 29 |
| Primate lentivirus group | 16 |
| Retro-transcribing viruses | 3 |
| Human herpesvirus | 4 |
| Homo sapiens | 3 |
| No Common Taxa | 2 |
| Simian-Human immunodeficiency virus | 1 |
| Danio rerio | 1 |
| Eukaryota | 1 |

**Table 4. Detection of CMV 5 infection.**

| **Organism** | **Unique organism hits** |
|---|---|
| Human herpesvirus 5 | 76 |
| Eutheria | 1 |
| Eukaryota | 1 |

### Example 4: Detection/identification of microorganism infection of subject cells: Detection of a Human papillomavirus (HPV) 18 infection of human cells.

### Infection of Hela cells with HPV and RNA sample preparation:

Human derived cervical carcinoma cells (HeLa cells) were infected with HPV 18. cDNA libraries of the samples were prepared as described in example 1. The cDNA libraries were then sequenced using the Illumina platform "Genome Analyzer IIx", according to manufacturer protocol.
The resulting sequencing obtained a total of 31,844,146 reads.

### Alignment and assembly:

The sequenced reads were clipped for the standard short RNA adapter using fast Xclipper. Discarding 964,062 reads which are shorter than 16 nt and 4,259,154 adapter only reads.

The reads were then aligned using Burrows-Wheeler Aligner (BWA) alignment software (default parameters) against the human genome (hg19) reference downloaded from UCSC index 11 (HPV). The alignment resulted with 26,026,437 mapped reads (97.76%) and 594,493 unmapped reads (2.24%). Aligning of the unmapped reads against the HPV 18 reference displayed 739 (0.124%) mapped reads.

### Categorization and identification

Utilizing "SRSA" on the unmapped reads resulted with 5 assembled sequences (contigs). The contigs were then aligned using NCBI's nucleotide megablast with a word size of 28, against the "All non-redundant GenBank CDS translations+PDB+SwissProt+PIR+PRF" database, with an inclusion threshold *E*-value of 0.01. Using an in-house developed software, only the highest ranking hits (with lowest E-value and highest total score) were included per query for the downstream analysis. The BLAST resulted with identification of HPV 18 having a unique organism hit 1 and Homo sapiens having a unique organism hit 1.

The results obtained herein above are of great importance and indicate that the method of the present invention can be implemented for clinical use. HPV can in some strains cause cancer and therefore utilizing the method of the present invention on clinical tissues can provide detection and/or identification of the exact infectious strain of HPV.

### Example 5: Detection/identification of microorganism infection of a subject using RandA software.

### Infection of Hela cells with HIV and mycoplasma and RNA sample preparation:

cDNA of mycoplasma and HIV 1 co-infected human T-cell culture samples were prepared as described in Example 1. Each sample was run twice on the deep sequencer. The sequenced data was analyzed using the ncRNA Read-and-Analyze (RandA) software (http://ibis.tau.ac.il/RandA). RandA is free-access software with a graphical user interface that carries through the essential steps in RNA transcriptome analysis after the acquisition of deep sequencing data. RandA generates a comprehensive analysis summary text including clipping, alignment and differential expression summaries and plots, depicting multiple alignments and post-clipping read length ratio.

### RandA software pipeline:

(i) Input and Output: entry of the deep sequencing output file for analysis. A condition name is assigned for each sequenced sample, allowing several samples to be assigned under the same condition (e.g several technical / biological replicates can be assigned under the same condition in order to infer the biological / technical variance). Reads deriving from short RNA transcripts (e.g miRNA) are usually included in the adapter sequence in addition to the transcript sequence itself. RandA software allows clipping this adapter sequence from the 3' ends of the reads and filtering out reads that were reduced to less than a set number of bases, utilizing, for example, FASTX Toolkit (http://hannonlab.cshl.edu/fastx_toolkit/).
(ii) Database preparation: For the purpose of ncRNA alignment and subsequent annotation, the extensive database of known ncRNA Rfam (v10.1) may be utilized. Rfam database contains almost 200,000 different organisms, and more than 1 million unique sequences for a variety of RNA species such as rRNA, tRNA, miRNA, cis-regulatory elements, snRNA, snoRNA, ribozymes and other documented non coding transcripts. Due to the high level of sequence diversity and size in Rfam, RandA allows the user to perform a variety of manipulations, creating a novel database, specifically tailored according to the relevant experimental needs. RandA enables to specify organisms, RNA families, and then collapse (join) transcripts that are either identical in sequence, or share the same description in order to further refine the database.
(iii) Analysis: RandA maps the reads against the newly formed database using a Burrows-Wheeler transform based alignment tool (BWA) which is summing the number of reads that mapped uniquely to each of the annotated ncRNA sequence. Due to the short read length produced by deep sequencing platforms, and the sequence similarity between the same families of ncRNAs, a significant amount of reads align to several different reference transcripts and their isoforms. Although these multiply aligned reads may add up to more than 50% of all mapped reads, they are usually excluded from the analysis, possibly leading to a biased and misleading expression profile. In order to circumvent this, RandA introduces multiple hits handling (reads mapped to more than one unique location or RNA sequence on the reference library) by implementing an expectation maximization based algorithm called SEQ-EM. SEQEM algorithm enables the inclusion of these multiple hits in the transcripts' final expression assessment, resulting in better analysis accuracy and power. The number of reads mapped to each transcript is then standardized according to its length and the initial total number of mapped reads based on the "reads per kilo-base per million" (RPKM) method. This allows the comparison between samples and a more precise transcript prioritization. The user may choose whether to continue and perform differential expression between the different conditions (samples), or to perform a transcript expression profiling. If the user selects to perform differential expression between two conditions, RandA reviews the number of samples assigned to each condition. If each condition has only one sample assigned to it (no replicates), a chi-squared test is performed to obtain P-values under the null hypotheses of no differential expression between samples. These P-values are further corrected according to the Bonferroni correction for multiple hypotheses testing. If one of the conditions has more then one sample assigned to it, transcript expression variance can be calculated. This allows a more accurate and powerful assessment of the difference in expression between conditions. For this purpose RandA employs DESeq, an "R"-based tool that performs differential expression analysis on deep sequencing data, and utilizes a negative binomial distribution model for variance estimation.

### Alignment and assembly:

The two small RNA samples extracted from a mycoplasma and HIV co-infected human T-cell culture were analyzed using RandA. The database of RandA was set to include all non-coding transcripts derived from either Homo-sapiens or bacteria. Other organisms were excluded. The newly generated database entailed 793,118 transcripts out of the original 2,756,313 registered in Rfam. Since Rfam transcript annotation is highly extensive, identical sequences might appear under different accessions numbers. However, RandA enables the user to reduce possible redundancy by collapsing transcripts either based on sequence or description identity (each might fit a different experimental question). Collapsing the combined human and bacteria database by sequence identity resulted in 394,240 unique sequences, a 50% reduction.

The sequence reads were clipped and aligned against the novel database. The alignment resulted in 88% and 54% of the mapped reads' unique alignment to the database in the uninfected and infected samples, respectively. The remaining reads that mapped to multiple locations were distributed using the inherent SEQ-EM algorithm to produce a new read count for each transcript. The counts in each deep sequencing run for each condition (sample) was analyzed using the DESeq tool, to produce a table describing the difference in expression between mapped transcripts (Table 5 herein). Table 5 demonstrates a partial representation of the output table produced by RandA. Base Mean 1 and 2 represent the mean normalized read count for each condition (sample). The Base mean is calculated using the DESeq tool which calculates a size factor for each condition by first constructing a "reference sample" by calculating for each gene, the geometric mean of the counts in all samples. Then, the quotient of the counts in the sample divided by the counts of the reference sample is calculated for each gene. Finally, the median of all the quotients are calculated to get a relative depth of the sample. This depth is used to normalize the read count for each gene in a sample. After normalization, a mean read count is calculated for each gene, for each condition.

**Table 5. Ten most differently expressed RNA transcripts.**

| **RNA Accession No.** | **RNA Description** | **Organism** | **Base Mean 1** | **Base Mean 2** | **Fold Change** | **Adjusted P-value** |
|---|---|---|---|---|---|---|
| AK292330.1/1-191 | U2 spliceosomal RNA | Homo sapiens (human) | 1.4 | 10384.93 | 7418.416 | 1.46E-30 |
| AE017243.1/17 8458-178387 | tRNA | Mycoplasma Hyopneumoniae J | 4.55 | 8283.436 | 1820.685 | 1.62E-29 |
| ABBA01175726 .1/642-527 | microRNA mir-689 | Homo sapiens (human) | 5.375 | 3934.626 | 732.047 | 7.13E-28 |
| ABSL01060990 .1/9336-9469 | U11 spliceosomal RNA | Homo sapiens (human) | 1.584 | 4291.487 | 2709.088 | 5.27E-27 |
| AE017332.1/33 7236-337309 | tRNA | Mycoplasma hyopneumoniae 232 | 0.508 | 2095.193 | 4121.295 | 2.12E-24 |
| AADD0100092 7.1/23641-23760 | U5 spliceosomal RNA | Homo sapiens (human) | 24.439 | 1749.023 | 71.566 | 1.60E-22 |
| AE017332.1/83 0793-830880 | tRNA | Mycoplasma hyopneumoniae 232 | 0.35 | 1572.036 | 4491.901 | 1.70E-22 |
| AADB0201003 4.1/410071-410401 | 7SK RNA | Homo sapiens (human) | 6.51 | 1665.874 | 255.914 | 4.19E-22 |
| EU714234.1/1-1496 | Bacterial small subunit ribosomal RNA | Mycoplasma hyorhinis | 1.4 | 980.269 | 700.25 | 4.02E-21 |
| AK292656.1/2-181 | U11 spliceosomal RNA | Homo sapiens (human) | 0.35 | 1082.402 | 3092.831 | 3.16E-20 |

### Categorization and identification:

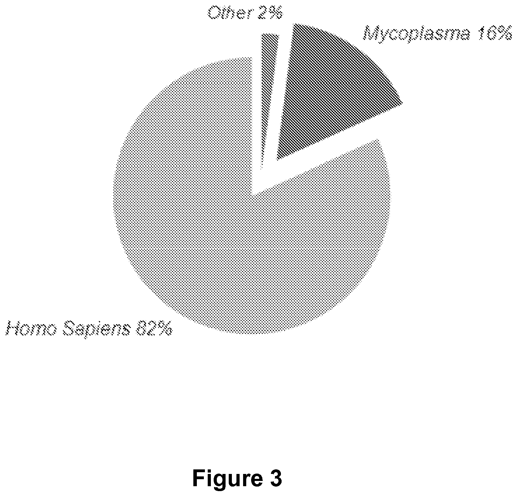

Using RandA, the significant different expression of mycoplasma derived transcripts of infected vs. uninfected samples was demonstrated. The analysis resulted in 2748 different RNA transcripts detected in at least one of the samples. 273 transcripts were considered significantly different in expression (P-Value<0.01), out of which 148 had a base mean count of over 100. Out of these 148 transcripts, 121 were human transcripts, 24 Mycoplasma and only 3 from other bacterial transcripts (Figure 3).

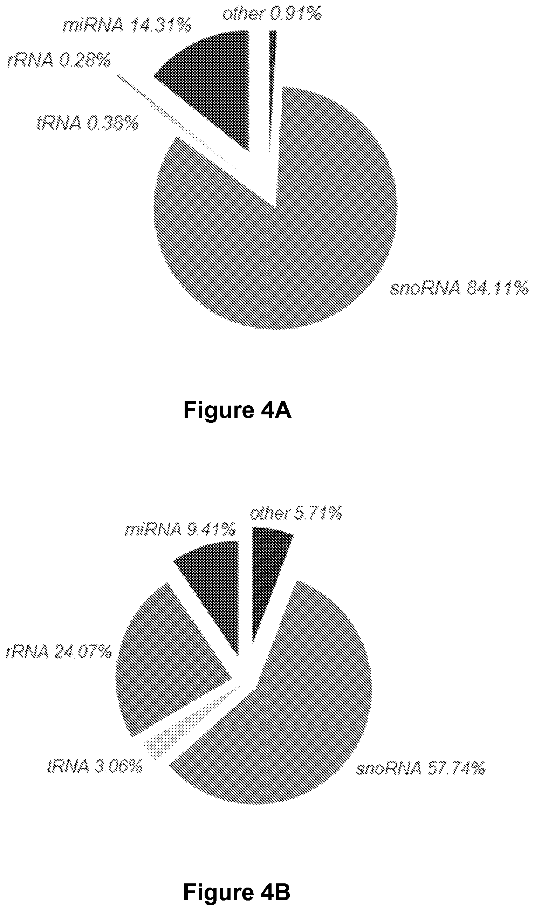

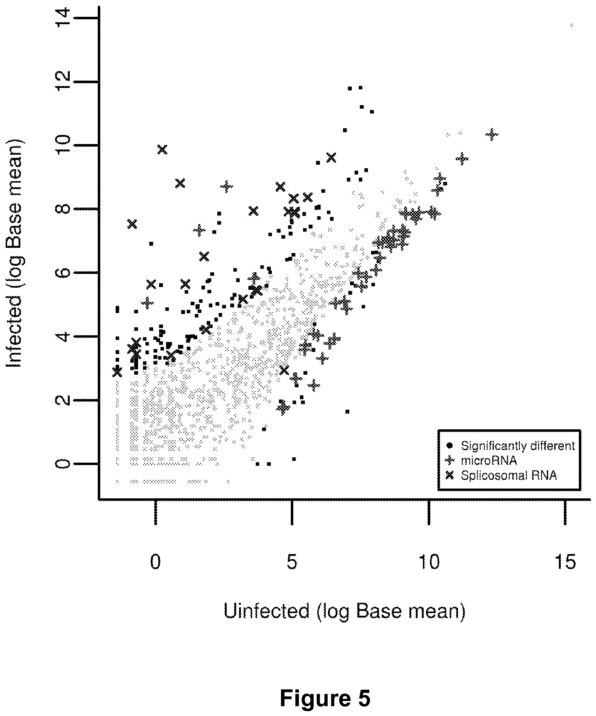

Running RandA with a defined database which includes viral transcripts did not result in any HIV related ncRNA transcript identification. This might be due to the scarcity of HIV related sequences in the sample or in the database. However, the human ncRNA transcript profile, of HIV infected (Fig. 4B) vs. uninfected (Fig. 4A) samples was analyzed. As can be seen in Figures 4A-B and Figure 5, a strong decrease in expression of miRNAs transcripts in the infected vs. the non infected samples (96%) was observed.

Decreased miRNA expression in infected human cells has been previously reported. Without wishing to be bound to theory or mechanism, this phenomenon can be attributed to the suspected Dicer-suppressive effect exerted by HIV-1 Tat protein and/or TAR RNA. The most significantly decreased miRNAs were further observed. When looking at the target genes of the most significantly decreased miRNAs in the infected sample, a significant enrichment (P-Value < 0.001) of genes in the Mitogen Activated Protein Kinase (MAPK) pathway was observed. The MAPK pathway is known to modulate and induce HIV infectivity. Thus, the decrease in MAPK pathway genes-targeting miRNAs could serve as a viral mechanism to induce pathway activity and subsequent increased infectivity. The analysis obtained supports HIV infection beyond the difference in miRNA expression levels. An enrichment of spliceosomal RNAs in the infected samples was observed (Figure 4B marked as "other" and Figure 5). Since HIV is known to disrupt the process of spliceosome assembly in the nucleus, enrichment of such splicosomal RNA fragments might support the presence of HIV in the sample. This could be a direct spliceosomal effect or via changing the stability of splicing factors. Additionally, 7SK RNA demonstrated a significant increase in expression in the infected sample (fold change >200; P-value<4*e-22), suggesting a cellular anti-viral defense mechanism given the disruption of HIV transcription.

### REFERENCES

Sébastien Pfeffer, Mariana Lagos-Quintana, Thomas Tuschl, (2005). Cloning of Small RNA Molecules. Current Protocols in Molecular Biology, Unit 26.4.
Cummings and Relman, Emerg. Infect. Dis. 6(5): 513 25 (2000).
MacConaill L, Meyerson M, Adding pathogens by genomic subtraction (2008)
Weber G, Shendure J, Tanenbaum DM, Church GM, Meyerson M, Identification of foreign gene sequences by transcript filtering against the human genome (2002).
Maiwald et al., Clin. Infect. Dis. 32(3): 457 463 (2001)
Xu Y, Stange-Thomann N, Weber G, Bo R, Dodge S, David RG, Foley K, Beheshti J, Harris NL, Birren B, Lander ES, Meyerson M, Pathogen discovery from human tissue by sequence-based computational subtraction, 2003.
Small RNA v1.5 Sample preparation Guide, Illumina, Sept. 2010 version.

## Claims

1. A method for the detection of infection of a mammal subject by a microorganism, the method comprising:
i) isolating a fraction of small RNA molecules having a length of about 10-100 nucleotides from a biological sample of the subject;
ii) aligning of nucleic acid sequences derived from the small RNA molecules against a reference database comprising the subject nucleic acid genome sequence;
iii) subtracting nucleic acid sequences that align with nucleic acid sequences in the reference database to obtain unmapped sequences which do not align with the nucleic acid sequences in the reference database and assembling the unmapped sequences to produce contigs having a length of about 10-500 nucleotides; and
iv) identifying the microorganism based on alignment of the contigs against databases comprising nucleic acid sequences.

2. The method of claim 1,
wherein the small RNA molecules are coding RNA molecules or fragments thereof; or wherein the small RNA molecules are non-coding RNA molecules.
wherein the small RNA molecules comprise piRNA, siRNA, miRNA, shRNA, snRNA, snoRNA, an RNA fragment of a larger RNA molecule, or any combination thereof.

3. The method of claim 1,
wherein the reference database comprising the subject nucleic acid genome sequence comprises a partial sequence of the subject nucleic acid genome; or
wherein the databases comprise: GenBank CDS (Coding sequences database), PDB (protein database), SwissProt database, PIR (Protein Information Resource) database, PRF (protein sequence) database, RNA families (Rfam) database, European Molecular Biology Laboratory (EMBL) Nucleotide Sequence database, or combinations thereof.

4. The method of claim 1, wherein the microorganism is selected from a bacterium, a virus, a fungi, a parasite, or combinations thereof.

5. The method of claim 4,
wherein the bacteria is selected from a gram negative bacteria, a gram positive bacteria, or both; or
wherein the virus is selected from dsDNA viruses, ssDNA viruses, dsRNA viruses, (+)ssRNA viruses (+)sense RNA, (-)ssRNA viruses (-)sense RNA, ssRNA-RT viruses (+)sense RNA with DNA intermediate in life-cycle, sDNA-RT viruses or combinations thereof; or
wherein the fungi is selected from yeast, mold, or both; or
wherein the parasite is selected from protozoa, helminths, or both.

6. The method of claim 1,
wherein the subject is a human or an animal; or
wherein the biological sample comprises a cell, a tissue, a body fluid, or combinations thereof; or
wherein at least one of the steps is performed using a computational system; or wherein the method is quantitative or qualitative.

7. The method of claim 1, further comprising a step of calculating a confidence score indicative of the confidence level that a microorganism has infected the subject.

8. The method of claim 7, wherein the confidence score is further indicative of the severity of the infection by the microorganism.

9. A system for detecting infection of a subject by a microorganism, the system comprising:
a sequencing device configured to determine nucleic acid sequences derived from small RNA molecules in the length of about 10-100 nucleotides, obtained from a biological sample of a mammal subject; and
a computing unit configured to:
a) align the nucleic acid sequences against a reference database comprising the subject nucleic acid genome sequence;
b) subtract nucleic acid sequences that align with nucleic acid sequences in the reference database to obtain unmapped sequences which do not align with the nucleic acid sequences in the reference database and assemble the unmapped sequences to produce contigs having a length of about 10-500 nucleotides; and
c) identify a microorganism based on alignment of the contigs against databases comprising nucleic acid sequences.

10. The system of claim 9,
wherein the small RNA molecules comprise piRNA, siRNA, miRNA, shRNA, snRNA, snoRNA, an RNA fragment of a larger RNA molecule, or any combination thereof; or
wherein the biological sample comprises a cell, a tissue, a body fluid, or combinations thereof.

11. The system of claim 9, wherein the microorganism is selected from a bacteria, a virus, a fungi, a parasite, or combinations thereof.

12. The system of claim 11,
wherein the bacteria is selected from a gram negative bacteria, a gram positive bacteria, or both; or
wherein the virus is selected from dsDNA viruses, ssDNA viruses, dsRNA viruses, (+)ssRNA viruses (+)sense RNA, (-)ssRNA viruses (-)sense RNA, ssRNA-RT viruses (+)sense RNA with DNA intermediate in life-cycle, sDNA-RT viruses or combinations thereof; or
wherein the fungi is selected from yeast, mold, or both.

13. The system of claim 11,
wherein the computing unit is further configured to calculate a confidence score indicative of the confidence level that a microorganism is infecting the subject; or
wherein the confidence score is further indicative of the severity of the infection by the microorganism; or
wherein the sequencing device is a deep sequencer.

14. A non-transitory computer-readable medium having stored therein instructions for detecting of infection of a mammal subject by a microorganism, wherein the instructions, when executed by the computer, cause the computer to:
a) align nucleic acid sequences derived from small RNA molecules having a length of about 10-100 nucleotides, obtained from a biological sample of a subject, against a reference database comprising the subject nucleic acid genome sequence;
b) subtract nucleic acid sequences that align with nucleic acid sequences in the reference database to obtain unmapped sequences which do not align with the nucleic acid sequences in the reference database and assemble the unmapped sequences to produce contigs having a length of about 10-500 nucleotides; and
c) identify a microorganism based on alignment of the contigs against databases comprising nucleic acid sequences.

## Patentansprüche

1. Verfahren zum Erkennen einer Infektion eines Säugetiersubjekts durch einen Mikroorganismus, das Verfahren umfasst:
i) Isolieren einer Fraktion von kleinen RNA Molekülen mit einer Länge von ungefähr 10-100 Nukleotiden aus einer biologischen Probe des Subjekts;
ii) Alignieren von Nukleinsäuresequenzen, die von den kleinen RNA Molekülen abstammen, gegen eine Referenzdatenbank, die die Nukleinsäuregenomsequenz des Subjekts umfasst;
iii) Subtrahieren der Nukleinsäuresequenzen, die mit Nukleinsäuresequenzen in der Referenzdatenbank alignieren, um unkartierte Sequenzen zu erhalten, die nicht mit den Nukleinsäuresequenzen in der Referenzdatenbank alignieren, und Zusammenfügen der unkartierten Sequenzen, um Contigs mit einer Länge von ungefähr 10-500 Nukleotiden zu erzeugen; und
iv) Identifzieren des Mikroorganismus basierend auf dem Alignieren der Contigs gegen Datenbanken, die Nukleinsäuresequenzen umfassen.

2. Verfahren nach Anspruch 1,
wobei die kleinen RNA Moleküle kodierende RNA Moleküle oder Fragmente davon sind; oder
wobei die kleinen RNA Moleküle nicht-kodierende RNA Moleküle sind; oder
wobei die kleinen RNA Moleküle piRNA, siRNA, miRNA, shRNA, snRNA, snoRNA, ein RNA Fragment eines größeren RNA Moleküls oder jegliche Kombination davon sind.

3. Verfahren nach Anspruch 1,
wobei die Referenzdatenbank, die die Nukleinsäuregenomsequenz des Subjekts umfasst, eine Teilsequenz der Nukleinsäuregenomsequenz des Subjekts umfasst; oder
wobei die Datenbanken umfassen: GenBank CDS (Coding sequences database), PDB (protein database), SwissProt Datenbank, PIR (Protein Information Resource) Datenbank, PRF (protein sequence) Datenbank, RNA Familien (Rfam) Datenbank, European Molecular Biology Laboratory (EMBL) Nucleotide Sequence Datenbank oder Kombinationen davon.

4. Verfahren nach Anspruch 1, wobei der Mikroorganismus ausgewählt ist unter einem Bakterium, einem Virus, einem Pilz, einem Parasiten, oder Kombinationen davon.

5. Verfahren nach Anspruch 4,
wobei das Bakterium ausgewählt ist unter einem Gram negativen Bakterium, einem Gram positive Bakterium oder beidem; oder
wobei das Virus ausgewählt ist unter dsDNA Viren, ssDNA Viren, dsRNA Viren, (+)ssRNA Viren (+)sense RNA, (-)ssDNA Viren (-)sens RNA, ssRNA-RT Viren (+)sense RNA mit DNA Intermediaten im Lebenszyklus, sDNA-RT Viren oder Kombinationen davon; oder
wobei der Pilz ausgewählt ist unter Hefe, Schimmel oder beidem; oder
wobei der Parasit ausgewählt ist unter Protozoen, Helminthen oder beidem.

6. Verfahren nach Anspruch 1,
wobei das Subjekt ein Mensch oder ein Tier ist; oder
wobei die biologische Probe eine Zelle, ein Gewebe, eine Körperflüssigkeit oder Kombinationen davon umfasst; oder
wobei wenigstens einer der Schritte unter Verwendung eines Computersystems durchgeführt wird; oder
wobei das Verfahren quantitative oder qualitative ist.

7. Verfahren nach Anspruch 1, ferner einen Schritt des Berechnens eines Konfidenzmaßes umfassend, das ein Konfidenzniveau anzeigt, dass ein Mikroorganismus das Subjekt infiziert hat.

8. Verfahren nach Anspruch 7, wobei das Konfidenzmaß ferner die Schwere der Infektion durch den Mikroorganismus anzeigt.

9. System zum Erkennen einer Infektion eines Subjekts durch einen Mikroorganismus, das System umfasst:
Eine Sequenziereinrichtung, die angepasst ist, Nukleinsäuresequenzen zu bestimmen, die von kleinen RNA Moleküle in der Länge von ungefähr 10-100 Nukleotiden abstammen und von einer biologischen Probe eines Säugetiersubjekts erhalten wurden; und
eine Computereinheit, angepasst, um:
a) die Nukleinsäuresequenzen gegen eine Referenzdatenbank zu alignieren, die die Nukleinsäuregenomsequenz des Subjekts umfasst;
b) Nukleinsäuresequenzen zu subtrahieren, die mit den Nukleinsäuresequenzen in der Referenzdatenbank alignieren, um unkartierte Sequenzen zu erhalten, die nicht mit den Nukleinsäuresequenzen in der Referenzdatenbank alignieren, und um die unkartierten Sequenzen zusammenzufassen, um Contigs zu erzeugen, die eine Länge von ungefähr 10-500 Nukleotide aufweisen; und
c) einen Mikroorganismus basierend auf dem Alignieren der Contigs gegen Datenbanken, die Nukleinsäuresequenzen umfassen, zu identifizierten.

10. System nach Anspruch 9,
worin die kleinen RNA Moleküle piRNA, siRNA, miRNA, shRNA, snRNA, snoRNA, ein RNA Fragment eines größeren RNA Moleküls oder jede Kombination davon umfassen; oder
worin die biologische Probe eine Zelle, ein Gewebe, eine Körperflüssigkeit oder Kombinationen davon umfasst.

11. System nach Anspruch 9, worin der Mikroorganismus ausgewählt ist unter einem Bakterium, einem Virus, einem Pilz, einem Parasiten, oder Kombinationen davon.

12. System nach Anspruch 11,
worin das Bakterium ausgewählt ist unter einem Gram negativen Bakterium, einem Gram positive Bakterium oder beidem; oder
worin das Virus ausgewählt ist unter dsDNA Viren, ssDNA Viren, dsRNA Viren, (+)ssRNA Viren (+)sense RNA, (-)ssRNA Viren (-)sens RNA, ssRNA-RT Viren (+)sense RNA mit DNA Intermediaten im Lebenszyklus, sDNA-RT Viren oder Kombinationen davon; oder
worin der Pilz ausgewählt ist unter Hefe, Schimmel oder beidem.

13. System nach Anspruch 11,
worin die Computereinheit ferner angepasst ist, ein Konfidenzmaßes zu berechnen, das ein Konfidenzniveau anzeigt, dass ein Mikroorganismus das Subjekt infiziert hat; oder
worin das Konfdenzmaß ferner die Schwere der Infektion durch den Mikroorganismus anzeigt; oder
worin die Sequenziereinrichtung ein Tiefensequenzierer ist.

14. Nicht vergängliches computerlesbares Medium, auf dem Instruktionen zum Erkennen einer Infektion eines Säugetiersubjekts durch einen Mikroorganismus gespeichert sind, wobei die Instruktionen, wenn sie durch einen Computer ausgeführt werden, den Computer dazu bringen:
a) Nukleinsäuresequenzen, die von kleinen RNA Moleküle mit einer Länge von ungefähr 10-100 Nukleotiden abstammen und von einer biologischen Probe eines Subjekts erhalten wurden, gegen eine Referenzdatenbank zu alignieren, die die Nukleinsäuregenomsequenz des Subjekts umfasst;
b) Nukleinsäuresequenzen zu subtrahieren, die mit den Nukleinsäuresequenzen in der Referenzdatenbank alignieren, um unkartierte Sequenzen zu erhalten, die nicht mit den Nukleinsäuresequenzen in der Referenzdatenbank alignieren, und um die unkartierten Sequenzen zusammenzufassen, um Contigs mit einer Länge von ungefähr 10-500 Nukleotide zu erzeugen; und
c) einen Mikroorganismus basierend auf dem Alignieren der Contigs gegen Datenbanken, die Nukleinsäuresequenzen umfassen, zu bestimmen.

## Revendications

1. Procédé de détection de l'infection d'un sujet mammifère par un micro-organisme, le procédé comprenant :
i) l'isolement d'une fraction de petites molécules d'ARN ayant une longueur d'environ 10 à 100 nucléotides à partir d'un échantillon biologique du sujet ;
ii) l'alignement des séquences d'acides nucléiques dérivées des petites molécules d'ARN par rapport à une base de données de référence comprenant la séquence génomique de l'acide nucléique du sujet ;
iii) la soustraction des séquences d'acides nucléiques qui s'alignent sur les séquences d'acides nucléiques dans la base de données de référence pour obtenir des séquences non cartographiées qui ne s'alignent pas sur les séquences d'acides nucléiques dans la base de données de référence et l'assemblage des séquences non cartographiées pour produire des contigs ayant une longueur d'environ 10 à 500 nucléotides ; et
iv) l'identification du micro-organisme d'après l'alignement des contigs par rapport aux bases de données comprenant les séquences d'acides nucléiques.

2. Procédé selon la revendication 1,
dans lequel les petites molécules d'ARN sont des molécules d'ARN codantes ou des fragments de celles-ci ; ou
dans lequel les petites molécules d'ARN sont des molécules d'ARN non codantes,
dans lequel les petites molécules d'ARN comprennent ARNpi, ARNsi, ARNmi, ARNsh, ARNsn, ARNsno, un fragment d'ARN d'une plus grosse molécule d'ARN, ou une quelconque combinaison de ceux-ci.

3. Procédé selon la revendication 1,
dans lequel la base de données de référence comprenant la séquence génomique de l'acide nucléique du sujet comprend une séquence partielle du génome de l'acide nucléique du sujet ; ou
dans lequel les bases de données comprennent : GenBank CDS (base de données de séquences codantes), PDB (base de données de protéines), la base de données SwissProt, la base de données PIR (ressources d'informations patients), la base de données PRF (séquence de protéines), la base de données de familles d'ARN (Rfam), la base de données du laboratoire européen de biologie moléculaire (EMBL) ou des combinaisons de celles-ci.

4. Procédé selon la revendication 1, dans lequel le micro-organisme est choisi parmi une bactérie, un virus, un champignon, un parasite ou des combinaisons de ceux-ci.

5. Procédé selon la revendication 4,
dans lequel la bactérie est choisie parmi une bactérie à Gram négatif, une bactérie à Gram positif ou les deux ; ou
dans lequel le virus est choisi parmi les virus d'ADNds, les virus d'ADNss, les virus d'ARNds, l'ARN sens (+) des virus d'ARNss (+) , l' ARN sens (-) des virus d'ARNss (-), l'ARN sens (+) des virus d'ARNss-RT avec l'intermédiaire d'ADN lors du cycle de vie, les virus d'ADNs-RT ou des combinaisons de ceux-ci ; ou
dans lequel le champignon est choisi parmi la levure, la moisissure ou les deux ; ou
dans lequel le parasite est choisi parmi les protozoaires, les helminthes ou les deux.

6. Procédé selon la revendication 1,
dans lequel le sujet est un humain ou un animal ; ou
dans lequel l'échantillon biologique comprend une cellule, un tissu, un liquide organique ou des combinaisons de ceux-ci ; ou
dans lequel au moins l'une des étapes est effectuée à l'aide d'un système informatique ; ou
lequel procédé est quantitatif ou qualitatif.

7. Procédé selon la revendication 1, comprenant en outre une étape de calcul d' un score de confiance indiquant le niveau de confiance selon lequel un micro-organisme a infecté le sujet.

8. Procédé selon la revendication 7, dans lequel le score de confiance indique également la gravité de l'infection par le micro-organisme.

9. Système de détection de l'infection d'un sujet par un micro-organisme, le système comprenant :
un dispositif de séquençage configuré pour déterminer les séquences d'acides nucléiques dérivées des petites molécules d'ARN dans la longueur d'environ 10 à 100 nucléotides, obtenues à partir d'un échantillon biologique d'un sujet mammifère ; et
une unité informatique configurée pour :
a) aligner les séquences d'acides nucléiques par rapport à une base de données de référence comprenant la séquence génomique de l'acide nucléique du sujet ;
b) soustraire les séquences d'acides nucléiques qui s'alignent sur les séquences d'acides nucléiques dans la base de données de référence pour obtenir des séquences non cartographiées qui ne s'alignent pas sur les séquences d'acides nucléiques dans la base de données de référence et assembler les séquences non cartographiées pour produire des contigs ayant une longueur d'environ 10 à 500 nucléotides ; et
c) identifier un micro--organisme d'après l'alignement des contigs par rapport aux bases de données comprenant les séquences d'acides nucléiques.

10. Système selon la revendication 9,
dans lequel les petites molécules d'ARN comprennent ARNpi, ARNsi, ARNmi, ARNsh, ARNsn, ARNsno, un fragment d'ARN d'une plus grosse molécule d'ARN, ou une quelconque combinaison de ceux-ci ; ou
dans lequel l'échantillon biologique comprend une cellule, un tissu, un liquide organique ou des combinaisons de ceux-ci.

11. Système selon la revendication 9, dans lequel le micro-organisme est choisi parmi une bactérie, un virus, un champignon, un parasite ou des combinaisons de ceux-ci.

12. Système selon la revendication 11,
dans lequel la bactérie est choisie parmi une bactérie à Gram négatif, une bactérie à Gram positif ou les deux ; ou
dans lequel le virus est choisi parmi les virus d'ADNds, les virus d'ADNss, les virus d'ARNds, l'ARN sens (+) des virus d'ARNss (+), l'ARN sens (-) des virus d'ARNss (-), l'ARN sens (+) des virus d'ARNss-RT avec l'intermédiaire d'ADN lors du cycle de vie, les virus d'ADNs-RT ou des combinaisons de ceux-ci ; ou
dans lequel le champignon est choisi parmi la levure, la moisissure ou les deux.

13. Système selon la revendication 11,
dans lequel l'unité informatique est en outre configurée pour calculer un score de confiance indiquant le niveau de confiance selon lequel un micro-organisme infecte le sujet ; ou
dans lequel le score de confiance indique également la gravité de l'infection par le micro-organisme ; ou
dans lequel le dispositif de séquençage est un séquenceur profond.

14. Support lisible par ordinateur non transitoire ayant des instructions de détection de l'infection d'un sujet mammifère par un micro-organisme stockées à l'intérieur, dans lequel les instructions, lorsqu'elles sont exécutées par l'ordinateur, demandent à l'ordinateur de :
a) aligner les séquences d'acides nucléiques dérivées des petites molécules d'ARN ayant une longueur d'environ 10 à 100 nucléotides, obtenues à partir d'un échantillon biologique d'un sujet, par rapport à une base de données de référence comprenant la séquence génomique de l'acide nucléique du sujet ;
b) soustraire les séquences d'acides nucléiques qui s'alignent sur les séquences d'acides nucléiques dans la base de données de référence pour obtenir des séquences non cartographiées qui ne s'alignent pas sur les séquences d'acides nucléiques dans la base de données de référence et assembler les séquences non cartographiées pour produire des contigs ayant une longueur d'environ 10 à 500 nucléotides ; et
c) identifier un micro-organisme d'après l'alignement des contigs par rapport aux bases de données comprenant les séquences d'acides nucléiques.
